# EUROPEAN PATENT APPLICATION

(11) **EP 2 634 255 A1**
(43) Date of publication of application: **04.09.2013**
(21) Application number: 11836528.7
(22) Date of filing: 02.08.2011
(51) Int. Cl.: C12N 15/29, C12N 15/87, A01H 5/00

(54) **COMPOSITION FOR PHYTOCHELATIN TRANSPORT**

(30) Priority: 28.10.2010 KR 20100106079
(71) Applicant: Postech Academy-industry Foundation, Gyeongsangbuk-do 790-784 (KR)
(72) Inventor: LEE, Youngsook, Pohang-si Gyeongsangbuk-do 790-825 (KR); MARTINOIA, Enrico, Pohang-si Gyeongsangbuk-do 790-784 (KR); PARK, Jiyoung, Seoul 153-701 (KR); SONG, Won-Yong, Pohang-si Gyeongsangbuk-do 790-390 (KR); MENDOZA-COZATL, David G., San Diego La Jolla, CA 92093-0116 (MX); SCHROEDER, Julian I., La Jolla, California 92093-0116 (US)
(74) Representative: Albutt, Jodie
(86) International application number: PCT/KR2011/005691
(87) International publication number: WO 2012/057436

(57) **Abstract**

A composition for transportation of phytochelatin including a DNA molecule encoding an ABCC (multidrug resistance-associated protein (MRP)) subfamily of ATP-binding cassette (ABC) transporter protein in a plant is provided. The composition for transportation of phytochelatin can be useful in accumulating and sequestering phytochelatin alone or in combination with a harmful substance by transporting the phytochelatin and/or harmful substance into the vacuole of a cell, thereby reducing a content of the toxic substance which moves from the root to the shoot of a plant. Therefore, the DNA molecule can be applied to develop a crop in which the toxic substance present in edible parts, such as leaves, stems and fruits, of the plant is reduced in content. Furthermore, the DNA molecule can be applied to inedible plants to develop plants useful for phytoremediation or phytoextraction due to an increase in accumulated amount of and resistance against arsenic, an arsenic compound and cadmium, thereby making it possible to achieve phytoremediation or phytoextraction in an environmentally friendly and economically clean manner.

## Description

### [Technical Field]

The present invention relates to a composition for transportation of phytochelatin including a DNA molecule encoding an ABCC (multidrug resistance-associated protein (MRP)) subfamily of ATP-binding cassette (ABC) transporter protein in a plant.

### [Background Art]

Arsenic (As) has been widely used in the fields of medicinal, industrial and agricultural applications. In the field of medicinal applications, arsenic (As) has been used to treat diseases such as syphilis, trypanosomiasis, or amoebic dysentery. In the field of agricultural applications, an arsenic-based herbicide such as disodium methane arsonate (DSMA) has been used to control weeds and harmful insects. However, arsenic (As) is a highly toxic environmental pollutant, and thus has been known to cause various health-related problems. In countries, such as China, Thailand and the US, in which mining operations are actively being conducted, as well as Bangladesh and India, arsenic (As) contamination of underground water has been reported. The contaminated underground water has become problematic since it is used as drinking water and agricultural water for several thousands of human beings.

Human beings are poisoned with arsenic (As) by drinking contaminated water or eating crops cultivated in the soil irrigated with the contaminated water. This poisoning is not restrictedly caused by arsenic (As), but generally caused by various toxic heavy metals and metalloids such as cadmium, mercury, and etc., which are accumulated in a human body through a food chain from the natural world to cause chronic damage to the brain, nerves, and bones. Also, since the metals and metalloids are not easily discharged from the human body, they cause problems in the human body for a long period of time. Furthermore, the contaminated environment and its damage last for generations. Therefore, it is very important to remove such toxic metals (or metalloids) from the environment.

In this regard, a living organism generally has a mechanism of mollifying toxicity of a harmful substance using a biomaterial or a transporter protein which binds to the harmful substance entering a human body. Genes contributing to resistance of such a living organism against the harmful substance may be used to clean up the environment contaminated with the harmful substances in a more economical and environmentally friendly manner than a method currently widely known in the art, such as physical, chemical and technological phytoremediation or phytoextraction. In particular, plants have advantages in that they do not destroy the preexisting ecosystem, are aesthetic and economically managed, and also exhibit new traits through simple expression of exogenous genes. Therefore, research is actively being conducted to improve plants for the purpose of use for phytoremediation or phytoextraction by implanting good genes into the plant. This is referred to as phytoremediation.

Accordingly, it is necessary to develop genetic information on plants taking part in absorbing more toxic heavy metals and metalloids and enhancing resistance against the toxic heavy metals and metalloids. Arsenic (As) is present in the form of a pentavalent ion, As(V), in the soil, and enters plant roots through protein transporting a phosphate having a structure similar to As(V). Then, the arsenic (As) is easily converted into a triavalent ion, As(III). Also, since most of the arsenic (As) is present in the form of As(III) under a reducing condition such as a rice field, it is absorbed into a plant by means of a membrane protein belonging to a family of aquaporins. In *Oryza sativa* L., As(III) is then discharged into the xylem by means of a silicon efflux transporter protein, Lsi2, so that arsenic (As) can be transported from the root to the shoot. In cells, As(III) is primarily bound to phytochelatin to be detoxified, and such a complex is finally transported into the vacuole to exhibit the resistance. Therefore, to force a plant to exhibit the resistance against the toxic heavy metals and metalloids, a role of a transporter protein for transporting a toxic metal (or metalloid)-phytochelatin complex into the vacuole is essentially required. However, a gene coding for a plant protein playing such a role remains to be found.

Phytochelatin (PC) functions in the first operation of detoxification by chelating metalloids and heavy metals such as arsenic (As), and the vacuole of a plant serves as a final detoxifying storage for heavy metal and arsenic (As). A detoxification procedure using PC is characteristic in plants and some other living organisms that produce PC, which distinguishes them from human beings and budding yeast which do not synthesize PCS (a gene coding for PC synthase) or PC. As such, there has been active research conducted for a long period of time to develop a PC-metal (or metalloid) transporter protein to sequester toxic metals (or metalloids) in the vacuole of a plant. However, both of a transporter protein taking part in transport of a heavy metal bound to PC into the plant vacuole and a transporter protein taking part in transport of As(III) bound to PC remain to be identified.

### [Disclosure]

### [Technical Problem]

Therefore, the present invention is designed to solve the problems of the prior art, and it is an object of the present invention to provide a composition for transportation of phytochelatin including a DNA molecule encoding an ABCC (multidrug resistance-associated protein (MRP)) subfamily of ATP-binding cassette (ABC) transporter protein in a plant.

It is another object of the present invention to provide a method of enhancing resistance of a plant against at least one selected from the group consisting of arsenic, cadmium and a compound thereof. Here, the method includes transforming a plant with the composition, and transporting at least one selected from the group consisting of arsenic, cadmium and a compound thereof in the form of a complex with the phytochelatin into a vacuole of the transformed plant.

It is still another object of the present invention to provide a method of transforming a plant with the composition and transporting phytochelatin, or a complex of the phytochelatin with at least one selected from the group consisting of arsenic, cadmium and a compound thereof into a vacuole of the transformed plant.

### [Technical Solution]

To achieve the above purpose, the present inventors have conducted research to screen members of the ABCC sub-family known as the multidrug resistance-associated protein (MRP) sub-family of ABC proteins to identify a candidate transporter protein considered to be associated with detoxification of a harmful substance in a plant. As a result, they have identified two ABC transporter proteins playing an important role in the detoxification of the harmful substance. More particularly, the present inventors have found that the two ABC transporter proteins are transporter proteins for phytochelatin (PC) or a PC complex in the vacuole required for resistance against the harmful substance such as arsenic, cadmium and/or a compound thereof. Accordingly, the present invention is completed based on these facts.

One aspect of the present invention provides a composition for transportation of phytochelatin, comprising a DNA molecule encoding an ABCC (multidrug resistance-associated protein (MRP))-subfamily of ATP-binding cassette (ABC) transporter protein in a plant.

Another aspect of the present invention provides a method of enhancing resistance of a plant against at least one selected from the group consisting of arsenic, cadmium and a compound thereof. Here, the method includes transforming a plant with the composition, and transporting at least one selected from the group consisting of arsenic, cadmium and a compound thereof in the form of a complex with phytochelatin into a vacuole of the transformed plant.

Still another aspect of the present invention provides a method of transforming a plant with the composition and transporting phytochelatin complex with at least one selected from the group consisting of phytochelatin, arsenic, cadmium and a compound thereof into a vacuole of the plant.

Hereinafter, the present invention will be described in further detail.

According to one exemplary embodiment of the present invention, a composition for transportation of phytochelatin, which includes a DNA molecule encoding an ABCC (multidrug resistance-associated protein (MRP))-subfamily of ATP-binding cassette (ABC) transporter protein in a plant, is provided.

A multidrug resistance-associated protein (MRP or ABCC)-subfamily of ATP-binding cassette transporter protein (hereinafter referred to as an ABCC-subfamily of ABC transporter protein) in a plant is a kind of an ABC transporter protein which serves to transfer various organic materials in the cytoplasm outside of a plasma membrane and transport the organic materials into the vacuole of the plant. The ABC transporter protein is present in various living organisms from prokaryotic organisms and liver cells of a human being, and transports extremely various materials.

The phytochelatin transported by the composition for transportation of phytochelatin according to one exemplary embodiment of the present invention may be phytochelatin itself (apo-PC₂) which is not bound to any component, or in the form of a complex of phytochelatin with at least one selected from the group consisting of zinc (Zn), copper (Cu), lead (Pb), silver (Ag), mercury (Hg), selenium (Se), arsenic (As), cadmium (Cd), and a compound thereof. More preferably, the phytochelatin may be in the form of a complex of phytochelatin with at least one selected from the group consisting of arsenic (As), cadmium (Cd), and a compound thereof.

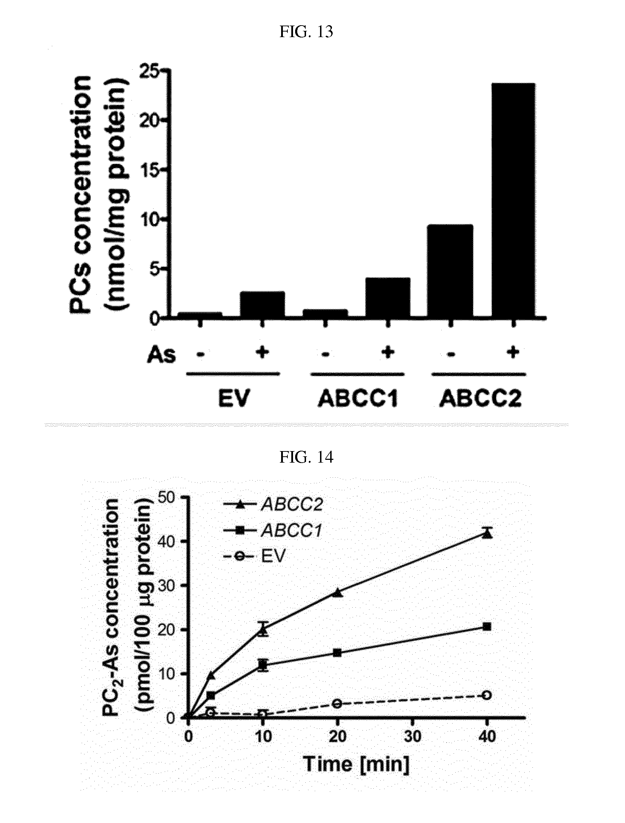

As shown in FIGS. 14 to 18, the transport of the phytochelatin itself (apo-PC₂) is no more effective than that of a PC₂-As complex, but the transport of apo-PC₂ by the ABC transporter protein provides the vacuole with apo-PC₂, which is required for stabilization of a toxic metal (or metalloid) transported into the vacuole by another transporter protein transporting an unbound metal and metalloid, and the apo-PC₂ may take place of, or be added, being effectively used to produce a high-molecular thiol-As complex having a high binding affinity to arsenic (As).

The final operation of detoxifying the harmful substance in cells is sequestration of a harmful substance into the vacuole, which depends mainly on phytochelatin (PC).

Phytochelatin (PC) is a metal-binding peptide whose synthesis is induced by other toxic metals as well as arsenic (As) or cadmium (Cd). PC serves to chelate harmful substances such as arsenic, cadmium and a compound thereof with a thiol group thereof, and sequester the chelate complex into the vacuole to protect components of cells from the harmful substances such as active metals (or metalloids).

Such PC-mediated detoxification is characteristic of some living organisms producing PC including all kinds of plants, for example, algae, moss, *Caenorhabditis elegans,* and fission yeast, which distinguishes them from human beings and budding yeast which do not synthesize PCS (a gene coding for PC synthase) or PC.

Especially, the present inventors have identified that detoxification of a harmful substance such as arsenic, cadmium and a compound thereof depends mainly on vacuolar sequestration of a toxic substance conjugated with phytochelatin in a plant.

Preferably, the plant includes *Arabidopsis thaliana, O. sativa, Nicotiana tabacum, Allium cepa, Daucus carota, Cucumis sativus, Brassica napus, Olea europaea, Ipomoea batatas, Solanum tuberosum, Brassica campestris* var. *pekinensis, Raphanus sativus, Lactuca sativa, Brassica oleracea* var. *italica, Petunia hybrida, Helianthus annuus, Brassica juncea* var. *integrifolia, Zoysia* spp., *Betula platyphylla* var. *japonica, Populus* spp., *Salix koreensis,* and *Betula schmidtii.* More preferably, A. *thaliana* or *O. sativa* are included.

In the present invention, the expression "DNA molecule encoding an ABCC subfamily of ABC transporter protein of a plant" encompasses meanings including genes that encode ABCC subfamily of ABC transporter protein of plants, a mutant, a derivative, a homologue and a fragment thereof, etc.

Preferably, the DNA molecules encoding the ABCC subfamily of ABC transporter proteins in a plant are genes encoding the ABCC subfamily of ABC transporter proteins, which may be genes encoding a PC transporter proteins present in the A. *thaliana* vacuole, for example, AtABCC1 (for example, NM_001036039.1 or NM_102777.2), AtABCC2 (for example, NM_129020.3), AtABCC11 (or AtMRP12) (for example, NM_102779.2), or AtABCC12 (or AtMRP13) (for example, NM_102778.3), and genes encoding proteins having high homology with AtABCC1 and AtABCC2 among the ABCC subfamily of ABC transporter proteins in *O. sativa* L., for example, OsABCC1 (for example, AJ535215.1), or OsABCC3 (or OsMRP11) (for example, NM_001072322.1).

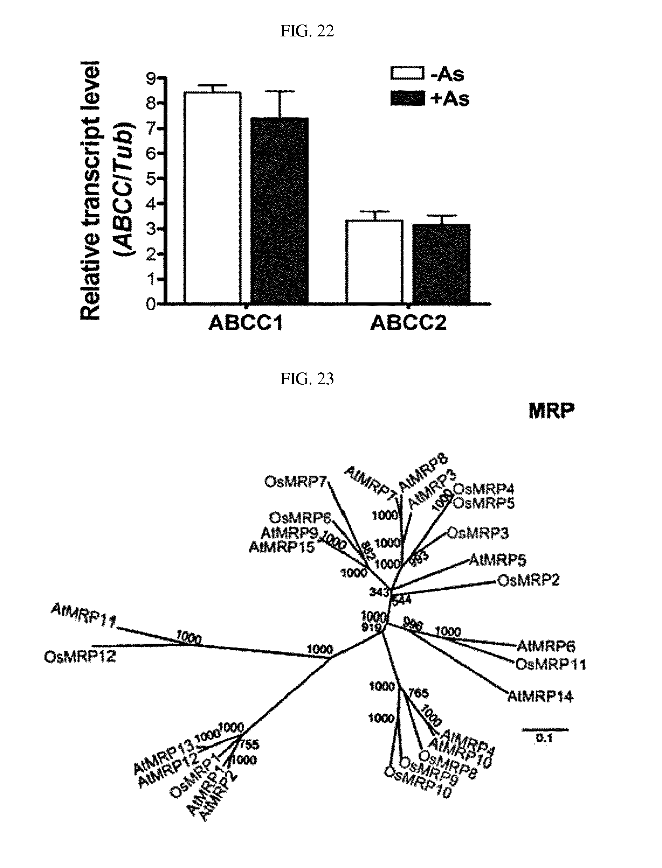

Transporter proteins encoded by the AtABCC1 and AtABCC2 of A. *thaliana* are highly conserved in an amino acid sequence, and belong to clade I of the ABCC sub-family and both exist in in a vacuole membrane. They transport arsenic, an arsenic-based herbicide or cadmium in the form of a complex with phytochelatin into the vacuole, and detoxify. Also, an amino acid sequence of a protein encoded by the OsABCC1 gene of *O. sativa* L. and AtABCC1 and AtABCC2 genes of *A*. *thaliana,* as shown in FIG. 23 has a sequence homology of 70% or more. More particularly, it has an identity of 70% or more and a similarity of 84% or more with the amino acid sequence of AtABCC1, and an identity of 72% or more and a similarity of 85% or more with the amino acid sequence of AtABCC2.

Therefore, the composition for transportation of phytochelatin according to the present invention has 70% or more of sequence homology with amino acid sequence of AtABCC1 or AtABCC2 protein and as it transports harmful substance in the form of a PC complex into the vacuole, it encompasses the meaning which includes DNA molecule that contributes in resistance and accumulation for harmful substance.

More preferably, the composition includes AtABCC1 (for example NM_001036039.1 or NM_102777.2), and AtABCC2 (for example NM_129020.3). The transporter proteins encoded by the AtABCC1 and AtABCC2 genes are phytochelatin transporter proteins that have been found in plants for a long period of time after discovery of phytochelatin (PC), and play an important role in PC transport activities in the vacuole of a plant cell including a mesophyll cell of *A*. *thaliana.* Therefore, the transporter proteins play a very important role in detoxification of various kinds of xenobiotic molecules, heavy metals, and metalloids including arsenic (As), which are bound to PC.

According to another exemplary embodiment of the present invention, a method of enhancing resistance of a plant against at least one selected from the group consisting of arsenic, cadmium and a compound thereof, which includes transforming a plant with the composition according to the present invention, and transporting at least one selected from the group consisting of arsenic, cadmium and a compound thereof in the form of a complex with phytochelatin into a vacuole of the transformed plant, is provided.

According to still another exemplary embodiment of the present invention, a method of transforming a plant with the composition according to the present invention and transporting phytochelatin, or a complex of the phytochelatin with at least one selected from the group consisting of arsenic, cadmium and a compound thereof into a vacuole of the transformed plant is provided.

The plant to be transformed includes all kinds of protoplasts, cells, plants, plant tissues, and plant organs.

The transformed plant includes a heterogeneous DNA sequence using a genetic engineering method, and is designed to be expressed in a plant cell, a plant tissue, a plant organ, or a plant and expresses the heterogeneous DNA sequence. A plant transformant may be prepared using a method known the related art and in this case, the known representative method is *A*. *tumefaciens-*mediated DNA transfer. More preferably, the recombinant *Agrobacterium* strain prepared using a method selected from the group consisting of electroporation, micro-particle injection, or a gene gun is introduced into a plant using a dipping method. A transformation system including a vector such as a viral vector (for example, obtained from *Cauliflower mosaic virus* (CaMV)) and a bacterial vector (for example, obtained from *Agrobacterium* spp.) may be widely used. After the prepared plant transformant is selected and/or screened, cells, tissues or organs of the transformed plant may be regenerated into intact plants using a known method. A transformation method and/or a regeneration technique may be properly selected, as apparent to those skilled in the art.

Preferably, the plant includes *A*. *thaliana, O. sativa, N. tabacum, A. cepa, D*. *carota, C*. *sativus, B. napus, O. europaea, I. batatas, S*. *tuberosum, B. campestris* var. *pekinensis, R. sativus, L. sativa, B. oleracea* var. *italica, P. hybrida, H. annuus, B. juncea* var. *integrifolia, Zoysia* spp., *B. platyphylla* var. *japonica, Populus* spp., *S*. *koreensis,* and *B. schmidtii,* and more preferably, *A*. *thaliana* or *O. sativa* are included.

Particularly, *O. sativa* L. generally grows in a water of a rice field which is a reducing environment. In this case, arsenic (As) present in the rice field exists mainly in the form of As(III). Improvement of sequestration and detoxification of arsenic (As) in the shoot and root of *O. sativa* L. results in a significant decrease in accumulation of arsenic (As) in rice grain, and prevent arsenic (As) from moving through the food chain, and the plant having improved resistance against and accumulation of the harmful substance may be highly useful in preventing severe health-related problems caused by intake of the harmful substance by reducing intake of the harmful substance for human beings who eat *O. sativa* L. as a staple food.

The cell is a prokaryotic cell or a eukaryotic cell, preferably, a cell of a plant selected from the group consisting of *A*. *thaliana, O. sativa, N. tabacum, A. cepa, D. carota, C. sativus, B. napus, O. europaea, I. batatas, S. tuberosum, B. campestris* var. *pekinensis, R. sativus, L. sativa, B. oleracea* var. *italica, P. hybrida, H. annuus, B. juncea* var. *integrifolia, Zoysia* spp., *B. platyphylla* var. *japonica, Populus* spp., *S*. *koreensis,* and *B. schmidtii.*

As described above, the composition for transportation of phytochelatin according to the present invention may be used to improve the resistance against or accumulation of harmful substances in plants, and simply clean sites contaminated with the harmful substances in an economic manner.

### [Advantageous Effects]

The composition for transportation of phytochelatin according to the present invention can be useful in accumulating and sequestering phytochelatin alone or in combination with a harmful substance by transporting the phytochelatin and/or harmful substance into the vacuole of a cell, thereby reducing a content of the toxic substance which moves from the root to the shoot of a plant.

Therefore, the composition for transportation of phytochelatin according to the present invention can be applied to develop a crop in which the toxic substance present in edible parts, such as leaves, stems and fruits, of the plant are reduced in content. Furthermore, the composition for transportation of phytochelatin according to the present invention can be applied to inedible plants to develop plants useful for phytoremediation or phytoextraction and increase the accumulated amount of and resistance against arsenic, an arsenic compound and cadmium, thereby making it possible to develop a plant that is useful in environmental cleanup (Phytoremediation or phyto-extraction) and results in environmentally friendly and economically clean manner effect.

### [Description of Drawings]

FIGS. 1 to 3 show high sensitivity of an *atabcc1 atabcc2* double knock-out mutant to cadmium (Cd), arsenic (As(V)), and an arsenic herbicide DSMA.
FIGS. 4 and 5 show the results obtained by comparing sensitivities of respective single mutants to the arsenic herbicide DSMA.
FIG. 6 shows the results obtained by comparing sensitivities of an *atabcc1 atabcc2* double knock-out mutant and a mutant, cad1-3, whose genes associated with PC synthesis are damaged, to arsenic (Ac).
FIGS. 7 and 8 show the weight and root length of a wild type (WT), an *atabcc1* knock-out mutant, an *atabcc2* knock-out mutant, and an *atabcc1 atabcc2* double knock-out mutant according to a concentration of arsenic (Ac).
FIG. 9 shows an *abcc1 abcc2* double knock-out homozygote line prepared by PCR and RT-PCR
FIG. 10 shows a homozygous knock-out genetic map of *abcc1-3.*
FIGS. 11 and 12 show that AtABCC1 and AtABCC2 have improved arsenic resistance and accumulation in budding yeast (SM7) expressing phytochelatin synthase (PCS).
FIG. 13 shows that an SM7 strain expressing AtABCC1 or AtABCC2 includes a much higher level of PC than an SM7 transformed with the AtABCC1 or AtABCC2.
FIG. 14 shows time-dependent absorption of PC₂-As into a microsomal vesicle of yeast (SM7).
FIG. 15 shows time-dependent absorption of PC₂-Cd into the microsomal vesicle of the yeast (SM7).
FIG. 16 shows comparison between transport activities of apo-PC₂ and PC₂-As, and inhibitory activities of transportation of PC₂-As by NH₄⁺ and vanadate.
FIG. 17 shows the results obtained by determining whether AtABCC2 transports arsenic (As) in a non-chelated form.
FIG. 18 shows concentration dependency of transport activities of PC₂-As (solid line) and apo-PC₂ (dotted line) in the microsomal vesicle in yeast (SM7) expressing AtABCC1 (square) or AtABCC2 (triangle).
FIG. 19 shows the results obtained by comparing transport activities of PC₂-As and apo-PC₂ into intact vacuoles separated from an *atabcc1 atabcc2* double knock-out plant and a WT plant.
FIG. 20 shows the total plant PC contents reflecting a pool of vacuoles and cytoplasm in the *atabcc1 atabcc2* double knock-out plant and the WT plant.
FIG. 21 shows the inhibitory activities of transportation of PC₂-As by NH₄⁺ and vanadate in A. *thaliana.*
FIG. 22 shows that AtABCC1 or AtABCC2 are not induced by arsenic (As) in A. thaliana.

FIG. 23 shows the sequence homology between amino acid sequences of proteins encoded by AtABCC1 and AtABCC2 genes and amino acid sequences of proteins encoded by OsABCC1 (OsMRP1), OsABCC3 (OsMRP11) genes of *O. sativa* L.

### [Best Mode]

Hereinafter, preferred embodiments of the present invention will be presented for a better understanding of the present invention. However, the description proposed herein is just an example for a better understanding, not intended to limit the scope of the invention.

**[Table 1]**

| *A. thaliana* ABCC (MRP) konkc-out mutation used in exemplary embodiments of the present invention | | |
|---|---|---|
| Name | Accession number | Knock-out number |
| *abcc1-3* | NM_102777.9 | Salk_017431 |
| *abcc2-2* | NM_129020.3 | Salk_127425 |
| *abcc3* | NM_112147.2 | WiscDsLox481-484C11 |
| *abcc4* | NM_130347.4 | Salk_093541 |
| *abcc5* | NM_100293.2 | Salk_002438 |
| *abcc6 mrp8* | NM_112148.3 | Salk_110544 |
| *abcc7* | NM_112149.3 | Salk_068478 |
| *abcc8* or *mrp6* | NM_113020.2 | Salk_110195 |
| *abcc9* | NM_115879.5 | Salk_087700 |
| *abcc10* or *mrp14* | NM_115776.3 | Salk_148625 |
| *abcc11-1* or *mrp12-1* | NM_102779.2 | Salk_137031 |
| *abcc12-2* or *mrp13-2* | NM_102778.3 | Salk_057394 |
| *abcc13* or *mrp11* | NM_126770.6 | Salk_044759 |
| *abcc14* or *mrp10* | NM_116135.2 | Salk_091563 |
| *abcc15* | NM_115961.1 | Salk_123843 |

**[Table 2]**

| Yeast lines used in exemplary embodiments of the present invention | | |
|---|---|---|
| Strains | Genotypes | Acquisition |
| DTY167 | *MA Ta ura3 leu2 his3 trp3 lys2 suc2 ycf::hisG* | Szczypka *et al.,* 1994 |
| YMM31 | *MA Ta, ura3-52 lys2-801 ade2-101 trp1-63 his3-200 leu2-1 ycf1::HIS3-MX6* | Dr. Karl Kuchler |
| Y11503 | *Mat a: his3D1; leu2D0; lys2D0; ura3D0; YLL015w::kanMX4* | EUROSCARF |
| YMM34 | *MA Ta ura3-52 lys2-801 ade2-101 trp1-Δ63 his3-Δ200 leu2-Δ1 yII048:: TRP1* | Dr. Karl Kuchler |
| SM4 | *ycf1:: His3, yhl035c::HIS3-MX6, yII015w::Kan-MX6, yll048c::TRP1-MX6* | Prepared in Example 2 |
| SM7 | *ycf1:: His3, yh1035c::HIS3-MX6, yII015w::Kan-MX6, yII048c::TRP1-MX6, TaPCS1:: cup1-1* | Prepared in Example 2 |

* The strains listed in Table 2 were acquired from:
- Szczypka et al., 1994 : Szczypka MS, Wemmie JA, Moye-Rowley WS, & Thiele DJ (1994) A yeast metal resistance protein similar to human cystic fibrosis transmembrane conductance regulator (CFTR) and multidrug resistance-associated protein. J. Biol. Chem. 269:22853-22857.
- Dr. Karl Kuchler: Medical University Vienna, Max F. Perutz Laboratories, Department of Medical Biochemistry, Vienna, Austria.
- EUROSCARF (EUROpean S. cerevisiae ARchive for Functional Analysis) : http://web.uni-frankfurt.de/fb15/mikro/euroscarf/

**[Table 3]**

| Primers used in exemplary embodiments of the present invention | | |
|---|---|---|
| SEQ ID NO | Primers | Sequences |
| 1 | AtABCC1-s | CCGCAGAAATCCTCTTGGTCTTGATG |
| 2 | AtABCC1-as | GTGAATCATCACCGTTAGCTTCTCTGG |
| 3 | AtABCC1,2P | ATCCGTGAACTCCGTTGGTATGTC |
| 4 | AtABCC1,2R | ACCACATGGTATGAAGTGATTGGC |
| 5 | AtABCC2RT-F | AGCGTGCCAAAGATGACTCACACCAC |
| 6 | AtABCC2RT-R | TACTTATCACGAAGAACACAACAGGG |
| 7 | His3MX6-YHL035F | GGGAACGGATCCCCTTATTATCCGAAATAATGGTTCATTTTGGG AAATCTGTTTAGCTTGC |
| 8 | His3MX6-YHL035R | GCTCTAGGCCCCCACTGTCACGACACATACTATAAAATATACCG CGTTTCTGCGCACTTAAC |
| 9 | YLL015F | ACCAAAACCAAGAAAGAAAGTCGTTCC |
| 10 | YLL015R | TGACTGAGAATACTGTTGTCATGTC |
| 11 | YLL048CF | AAAACTGCTCCTGATCTCAAGCCAC |
| 12 | YLL048CR | AATGTTCTCTCGAGCTTCCAAGGCC |
| 13 | TaPCS1::cup1-1F | ATAGATATTAAGAAAAACAAACTGTACAATCAATCAATCAATCA TCACATAAAATGGAGGTGGCGTCGCTG |
| 14 | TaPCS1::cup1-1R | TTTAAAAATTAAAAACAGCAAATAGTTAGATGAATATATTAAA GACTATTCGTTTCAAGTGAGCAGATTGTCGCAG |
| 15 | Salk_017431 LP | TTCACTGTATCCATTGCCG |
| 16 | Salk_017431 RP | TTACCGGCTTAGGTCTGCACTG |
| 17 | Salk_127425 LP | GTGTTCGGGGTGCTATGTGAA |
| 18 | Salk_127425 RP | CTGTAAAGGGAACATAAGGAC |

### Example 1: Hypersensitiveness test of atabcc1 and atabcc2 double knock-out mutant on arsenic, arsenic herbicide or cadmium

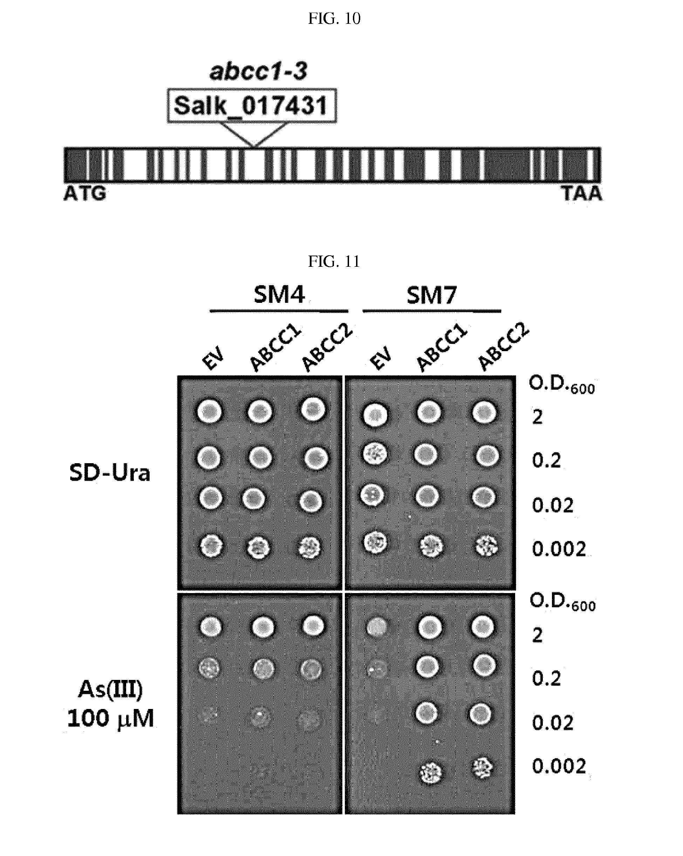

A total of 15 homozygous knock-out lines for an A. *thaliana* ABCC gene were separated, and the mutants were grown in the presence of arsenic, an arsenic-containing herbicide, and cadmium. Apart from *abcc2-2, abcc11-1,* and *abcc12-2* kindly provided by Dr. Markus Klein (Frelet-Barrand A, et al. (2008) Comparative mutant analysis of Arabidopsis ABCC-type ABC transporters: AtMRP2 contributes to detoxification, vacuolar organic anion transport and chlorophyll degradation. Plant Cell Physiol 49:557-569), all the abcc knock-out mutants were acquired from the Salk Genomic Analysis Laboratory (http://signal.salk.edu/). The accession numbers and knock-out numbers of all the 15 *abcc* mutants are listed in Table 1. Among these, a new mutant allele, Salk_017431, of ABCC1 was used in this experiment, and named *abcc1-3* after the previously studied alleles, *abcc1-1* and *abcc1-2.* A homozygous knock-out gene of *abcc1-3* was separated by RT-PCR using primers AtABCC1-s (SEQ ID NO: 1) and AtABCC1-as (SEQ ID NO: 2) (FIG. 10). Primer sequences used are listed in Table 3.

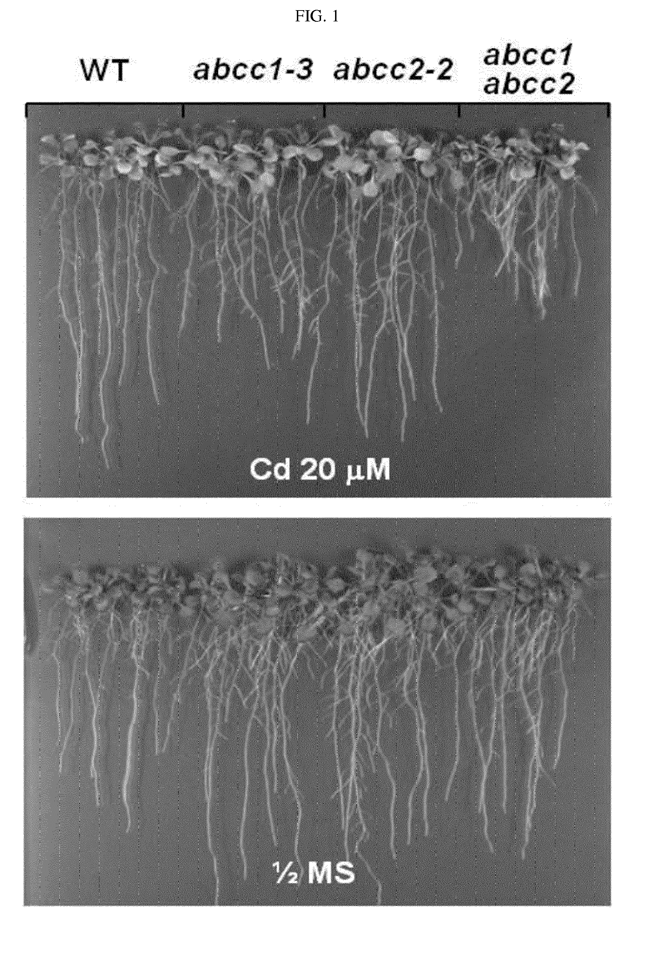

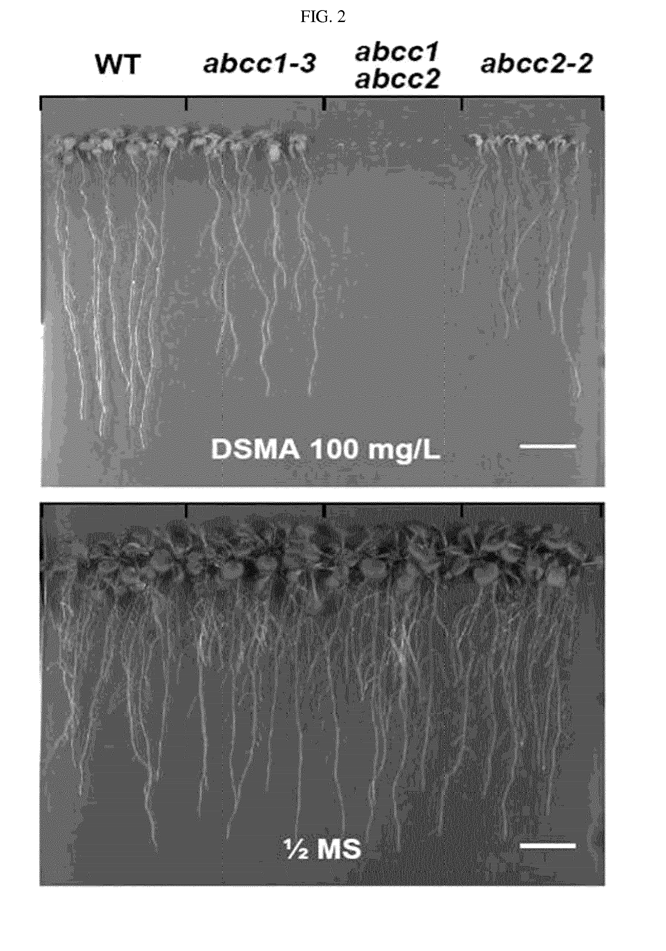

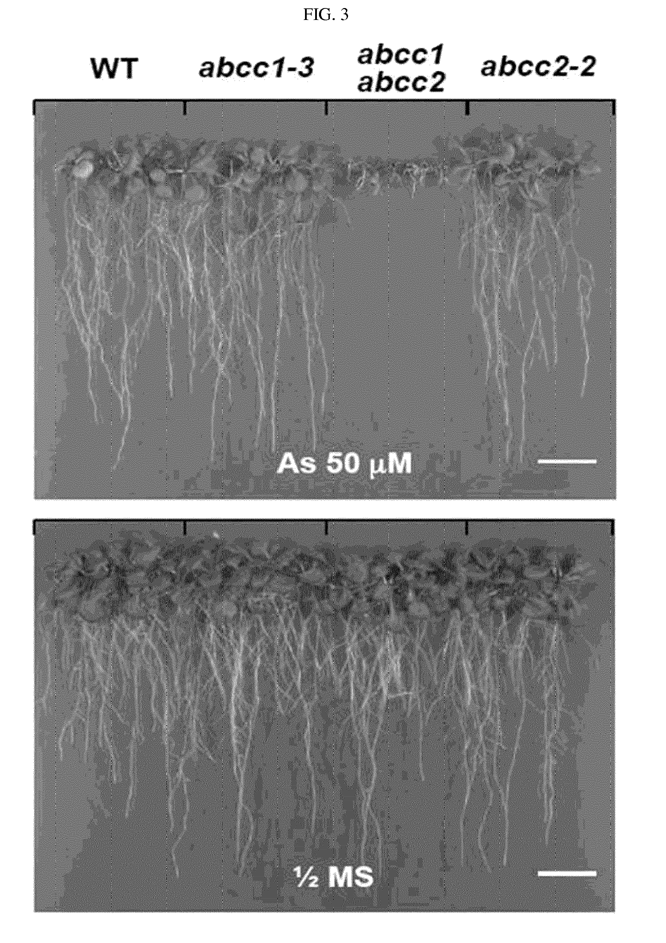

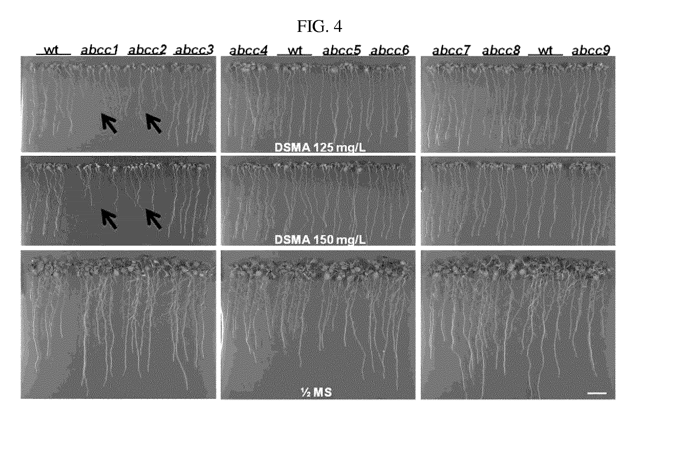

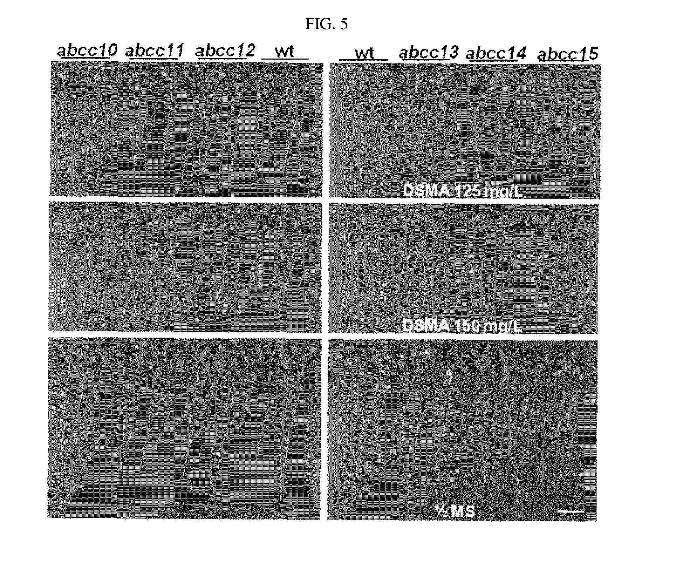

Seeds of WT and transformed (ecotype Columbia-0) A. *thaliana* strains were grown for 16 days in a 1/2 concentration MS (Murashige T, Skoog F (1962) A revised medium for rapid growth and bioassays with tobacco tissue cultures. Physiol Plant 15:473-493, Duchefa) agar plate supplemented with 1.5% sucrose in the presence or absence of arsenate ((As(V), Na₂HAs0₄)) (FIG. 3), an arsenic-based herbicide, disodium methane arsonate (DSMA) (Supleco, http://www.sigmaaldrich.com) (FIG. 2), or cadmium (FIG. 1) under an environment in which a light cycle was controlled for 16 hours and a dark cycle was controlled for 8 hours at 22 °C/18 °C. No As(V)- or Cd-sensitive atabcc1 or atabcc2 single knock-out mutants are observed, but two single-deleted *atabcc1* and *atabcc2* mutants were not well grown, compared with the wild-type strain (FIG. 1 to FIG. 10, scale bar = 1 cm).

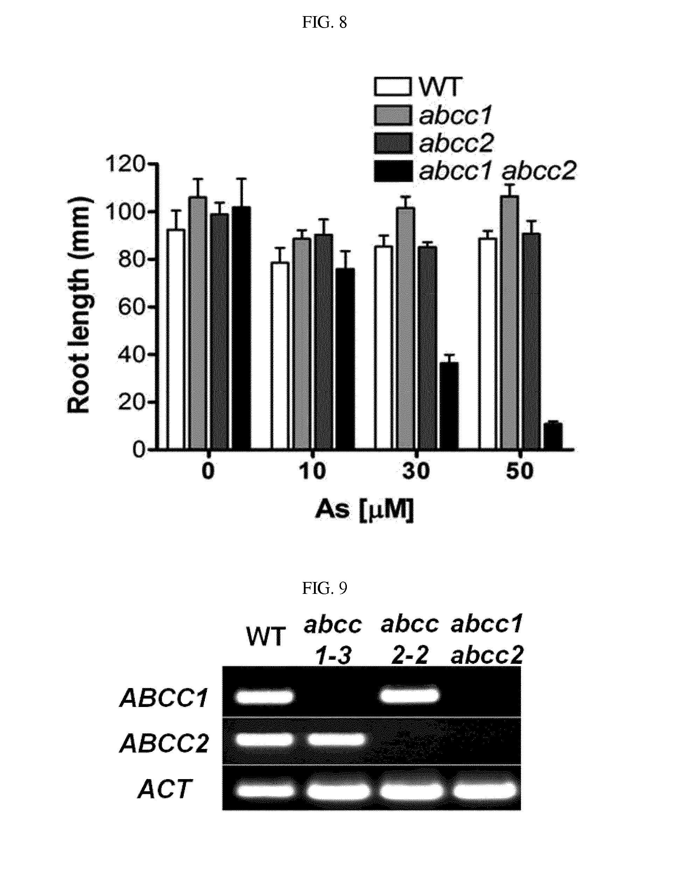

To determine whether the relatively proper arsenic or cadmium sensitivity was due to the function overlapping between the two ABC transporter proteins, an *atabcc1 atabcc2* double knock-out strain was prepared. An *abcc1 abcc2* double knock-out homozygote line was selected by genomic PCR using primers, AtABCC1,2F (SEQ ID NO: 3) and AtABCC1,2R (SEQ ID NO: 4), which contain generic sequence fragments of AtABCC1 and AtABCC2 (for 5 minutes at 95 °C → (35 cycles repeated) for 50 seconds at 95 °C → for 40 seconds at 58 °C → for 30 seconds at 72 °C (finished) → for 10 minutes at 72 °C), and RT-PCR using primers, AtABCC1-s (SEQ ID NO: 1) and AtABCC1-as (SEQ ID NO: 2), for AtABCC1 and primers, AtABCC2RT-F (SEQ ID NO: 5) and AtABCC2RT-R (SEQ ID NO: 6), for AtABCC2 (for 5 minutes at 94 °C → (25 cycles repeated) for 50 seconds at 94 °C → for 40 seconds at 58 °C → for 30 seconds at 72 °C (finished) → for 10 minutes at 72 °C) (FIG. 9). While a difference in growth between the wild type and the double knock-out mutant was not observed under control conditions, growth of the *atabcc1 atabcc2* knock-out line on a plate containing a low content of As(V) or Cd and a plate including DSMA was dramatically inhibited, compared with the wild type (FIGS. 1 to 10).

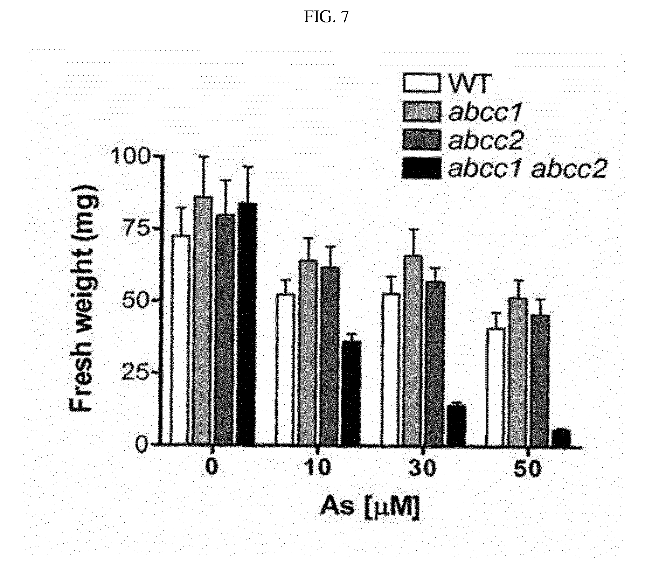

To analyze the conditions and the phenotypes of the lines, 7 plants were collected and weighed, and root lengths of the plants were measured (FIGS. 7 and. 8). Fresh weights and root lengths of each genotype of plants grown in a 1/2 MS medium supplemented with 0 to 50 µM As(V) are shown in FIGS. 7 and 8, respectively. Average ± standard error of estimation (s.e.m.) (n (population) = 16 obtained from 4 independent experiments to measure fresh weights of the plants; and n (population) = 332 to 336 obtained from 3 independent experiments to measure root lengths of the plants). * P < 0.04, ** P < 0.01 (students t-test).

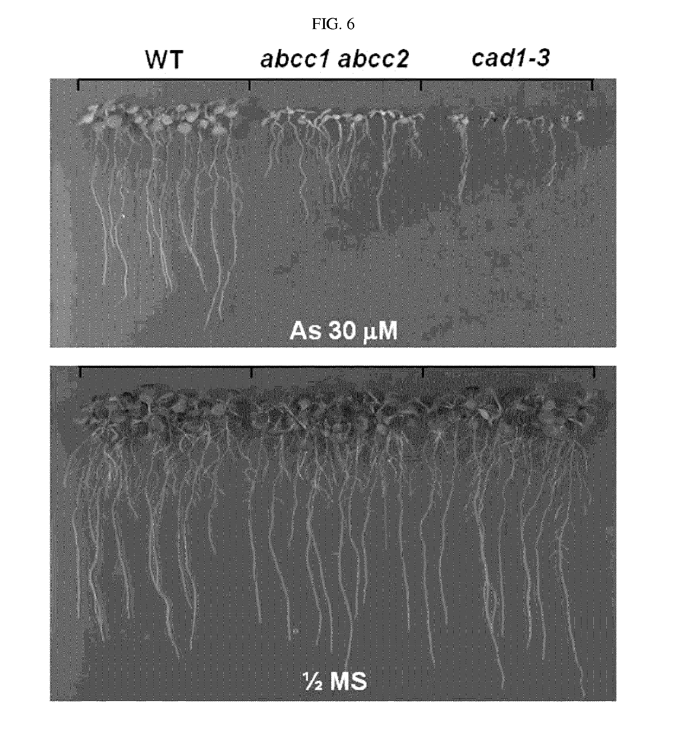

For relationship anlysis between ABCC1 and 2 and PCS1, *cad1-3* which was a knock-out mutant of phytochelatin synthase 1 (PCS1) (Howden R, Goldsbrough PB, Andersen CR, Cobbett CS (1995) Cadmium-sensitive, cad1 mutants of A. thaliana are phytochelatin deficient. Plant Physiol 107:1059-1066) was used. The sensitivity of the *atabcc1 atabcc2* knock-out line observed was similar to that of the *cad1-3* whose genes associated with PC synthesis were damaged (FIG. 6). As described above, it was revealed that the *atabcc1 atabcc2* double knock-out and the similar phenotype of deficient mutant indicates that a function of a transporter to sequester a PC₂-As complex into the vacuole is as important as PC synthesis for detoxification of arsenic (As).

### Example 2: Test on whether AtABCC1 and AtABCC2 in budding yeast improve arsenic resistance and accumulation in the presence of PCS1 (phytochelatin synthase 1)

To determine whether the AtABCC1 and AtABCC2 give resistance against arsenic (As), PCR products of yhl035c::HIS3-MX6, yll015w::Kan-MX6 and y11048c::TRP1-MX6 were inserted into homologous sites (YHL035c, YLL015w, and YLL048c) of a Cd-sensitive mutant, DTY167 yeast gDNA, by means of homologous recombination and prepare budding yeast mutant SM4 mutant that is deficient of YCF1 and three other vacuole ABCC-type ABC transporter proteins, YHL035c, YLL015w, and YLL048c, and the transporter proteins AtABCC1 and AtABCC2 were expressed in the prepared SM4.

To amplify a DNA fragment for homologous recombination, the following primers and genomic DNA obtained from a single null mutant were used: Primers, His3MX6-YHL035F (SEQ ID NO: 7) and His3MX6-YHL035R(SEQ ID NO: 8), for yh1035c::HIS3-MX6, genomic DNA obtained from a YMM31 strain, primers, YLL015F (SEQ ID NO: 9) and YLL015R (SEQ ID NO: 10) for yll015w::Kan-MX6, DNA obtained from a Y11503 strain, primers, YLL048CF (SEQ ID NO: 11) and YLL048CR (SEQ ID NO: 12), for yll048c::TRP1-MX6, and DNA obtained from a YMM34 strain.

SM4 cells were grown for 2 days in a 1/2 concentration SD-Ura medium (Qbiogene) supplemented with 100 µM As(III). O.D.600 was an optical density of a yeast suspension measured at 600 nm, as indicated on a plate for the first time.

The arsenic resistance test revealed no clear difference in genotype between the SM4 yeast lines expressing AtABCC1 or AtABCC2, and the transformants showed slight increase in resistance compared with the void vector control (pNEV-ura: Sauer N & Stolz J (1994) SUC1 and SUC2: two sucrose transporters from A. thaliana; expression and characterization in baker's yeast and identification of the histidine-tagged protein. Plant J 6:67-77) (FIG. 11). These results indicate that, unlike in A. *thaliana,* AtABCC1 and AtABCC2 could not mediate the arsenic resistance in yeast. These experiments showed that detoxification of arsenic (As) in plants depend mainly on PC.

Therefore, the present inventors tested whether the AtABCC1 and AtABCC2 gave the arsenic resistance by transporting a PC₂-As complex. First, a yeast mutant expressing PC synthase, TaPCS1, from wheat *(Triticum aestivum)* under the SM4 background was prepared, and was named "SM7."

The SM7 mutant was prepared by inserting TaPCS1(AF093752) (Clemens S, Kim EJ, Neumann D, & Schroeder JI (1999) Tolerance to toxic metals by a gene family of phytochelatin synthases from plants and yeast. EMBO J 18:3325-3333.) into CUP1-1 exiting in an SM4 strain so as to express wheat PC synthase, TaPCS1, under a CUP1-1(NM_001179183.1) promoter. That is, a TaPCS1::cup1-1 gene was amplified using primers, TaPCS1::cup1-1F(SEQ ID NO: 13) and TaPCS1::cup1-1R(SEQ ID NO: 14), and a template, TaPCS 1 cDNA(AF093752), and SM4 yeast was then transformed using homologous recombination.

To express AtABCC1 in yeast, AtABCC1 cDNA (NM_102777.2) was subcloned into a SacII/SalI restriction site of a vector, pGEM-T easy (Promega Corp.), from pBsc-MRP1 (Geisler M, et al. (2004) Arabidopsis immunophilin-like TWD1 functionally interacts with vacuolar ABC transporters. Mol Biol Cell 15:3393-3405). Thereafter, pNEV-AtABCC1 was prepared by inserting an oligonucleotide NotI linker into a BstXI restriction site of a pGEM-T easy vector in which a total of cDNA digested with NotI/NotI restriction enzymes was subcloned into a NotI restriction site of pNEV. Yeast cells SM4 and SM7 were transformed with the pNEV void vector (Sauer N & Stolz J (1994) SUC1 and SUC2: two sucrose transporters from A. thaliana; expression and characterization in baker's yeast and identification of the histidine-tagged protein. Plant J 6:67-77), pNEV-AtABCC1 and pYES3-AtABCC2 (Lu YP, et al. (1998) AtMRP2, an Arabidopsis ATP-binding cassette transporter able to transport glutathione S-conjugates and chlorophyll catabolites: functional comparisons with AtMRP1. Plant Cell 10:267-282) using a lithium acetate method (Ito H, Fukuda Y, Murata K, Kimura A (1983) Transformation of intact yeast cells treated with alkali cations. J Bacteriol 153:163-168), and screened on a uracil-deficient synthetic dextrose agar plate (SD-Ura) (Qbiogene). For arsenic resistance tests, SM4 and SM7 yeast lines expressing the given proteins, AtABCC1 and AtABCC2, were grown at 30 °C for 2 days in a 1/2 concentration uracil-deficient minimal medium (1/2 SD-Ura) (Qbiogene) in the presence or absence of 100 µM As(III).

Next, the yeast lines were used to express the AtABCC1 or AtABCC2. When the yeast lines were grown in an arsenic-free control medium, for example, a 1/2 concentration uracil-deficient minimal medium (1/2 SD-Ura, Qbiogene), the yeast lines expressing either the void vector or the ABC transporter protein were observed, but no significant difference between transformed SM7 cells was observed. However, when the yeast cells were exposed to 100 µM As(III), the yeast cells expressing the AtABCC1 or AtABCC2 showed remarkably better growth than the control (EV = void vector), and thus exhibited improved resistance against arsenic (As) to a concentration of 100 µM (FIG. 11).

To measure a content of arsenic (As) in yeast cells, SM7 yeast cells transformed with a void vector (pNEV-Ura), pNEV-AtABCC1 or pYES3-AtABCC2 were grown for 12 hours in a 1/2 SD-Ura medium supplemented with 100 µM As(III). The same number of cells from each strain was used to measure the content of arsenic (As). The yeast cells were washed twice with a 10 mM K⁺-phosphate buffer (pH 7), and then washed once with cold water. Thereafter, the yeast cells were digested with 11 N HNO₃ at 100 °C for 6 hours. After the samples were completely digested, the samples were diluted with distilled water, and a content of arsenic (As) was analyzed using an ICP-MS spectrometer (ELAN DRC e, Perkin Elmer, http://www.perkinelmer.com/).

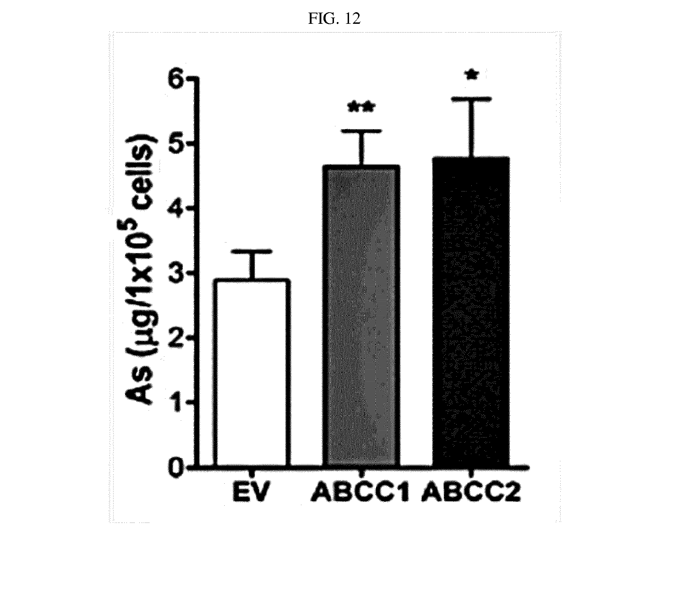

The yeast cells were grown for 12 hours in a 1/2 concentration SD-Ura medium supplemented with 100 µM As(III). Average ± standard error of estimation (n = 21 obtained from 7 independent experiments). * P < 0.03, ** P < 0.005 (students t-test). Under such conditions, SM7 yeast cells expressing the AtABCC1 or AtABCC2 accumulated a larger amount of arsenic (As) compared to the corresponding control (EV = void vector) (FIG. 12).

To measure a level of PC, S. *cerevisiae* strains, SM4 and SM7, containing a void plasmid (pNEV-Ura: Sauer N & Stolz J (1994) SUC1 and SUC2: two sucrose transporters from A. thaliana; expression and characterization in baker's yeast and identification of the histidine-tagged protein. Plant J 6:67-77), AtABCC1 or AtABBC2 were grown in an SD-Ura medium (Qbiogene) including 100 µM CuSO₄ until a value of OD₆₀₀ₙₘ reached 1.0. The S. *cerevisiae* cells were exposed to 100 µM As₂O₃ for 20 hours, and centrifuged for 15 minutes at a rotary speed of 2,000 g and harvest and then, was homogenized with glass beads according to a method disclosed in Vande Weghe JG, Ow DW (2001) Accumulation of metal-binding peptides in fission yeast requires hmt2+. Mol Microbiol 42:29-36. The cell extract obtained as described above was used for PC analysis according to a method disclosed in Mendoza-Cozatl DG, et al., (2008) Identification of high levels of phytochelatins, glutathione and cadmium in the phloem sap of B. napus. A role for thiol-peptides in the long-distance transport of cadmium and the effect of cadmium on iron translocation. Plant J 54:249-259. In the presence of arsenic (As), the SM7 strain expressing the AtABCC1 or AtABCC2 contained a remarkably higher level of PC, compared with the non-transformed SM7 strain (FIG. 13).

The results of this experiment, revealed that, since the AtABCC1 and AtABCC2 could mediate sequestration of a PC₂-As complex into the vacuole, the yeast line producing PC enables to treat arsenic (As). Since the growth of SM4 and SM7 (EV) yeast lines weren't that different on a medium supplemented with 100 µM As(III), the non-homologous recombination analysis also suggested that arsenic (As) could not be detoxified only by chelating of arsenic (As) by PC, and a transporter protein for a

PC₂-As complex was essentially required.

### Example 3: Test on whether AtABCC1 and AtABCC2 act as phytochelatin transporter protein

SM7 yeast cells transformed with a void vector (pNEV-Ura) (Sauer N & Stolz J (1994) SUC1 and SUC2: two sucrose transporters from A. thaliana; expression and characterization in baker's yeast and identification of the histidine-tagged protein. Plant J 6:67-77), pNEV-AtABCC1 or pYES3-AtABCC2 (Lu YP, et al. (1998) AtMRP2, an Arabidopsis ATP-binding cassette transporter able to transport glutathione S-conjugates and chlorophyll catabolites: functional comparisons with AtMRP1. Plant Cell 10:267-282) were grown in an SD-Ura liquid medium (Qbiogene) at 30 °C for 4 hours, and then centrifuged for 15 minutes at a rotary speed of 2,000 g to harvest a cell pellet. All the cell lines were incubated in a YPD medium (USB, Duchefa) at 30 °C for 30 minutes, centrifuged for 15 minutes at a rotary speed of 2,000 g to harvest a cell pellet, and the cell pellet was then disassembled with lyticase (1,000 units/g fresh weight of cells, Sigma). Then, a microsomal vesicle was separated from the cell extract using a method disclosed in Tommasini R, et al,, (1996) The human multidrug resistance-associated protein functionally complements the yeast cadmium resistance factor 1. Proc Natl Acad Sci USA 93:6743-6748, and used to perform a transportation experiment.

Before starting the transportation experiment, 10 µl of the thawed microsomal vesicle (100 µg of a protein) was resuspended in 90 µl of a transporting buffer, and kept frozen for 5 minutes before incubated at 25 °C. In the times indicated in the drawings, reaction products were diluted with a very cold washing buffer (100 mM KCl and 25 mM Tris-Mes (pH 7.4)) until a final volume reached 1 ml, and instantly filtrated under a vacuum condition through a nitrocellulose filter (Millipore, http://www.millipore.com/) with fine pores having a diameter of 0.45 µm. The resulting filtrate was washed with 2 ml of a washing buffer (100 mM KCl and 25 mM Tris-Mes (pH 7.4)).

For a PC₂ transportation experiment, ³⁵S-PC₂ of 25 nCi (920 Bq) was used as a tracer in all the analyses. The ³⁵S-PC₂ was synthesized using a recombinant AtPCS 1-6xHis protein (AF085230) and 35S-GSH (60 uCi, 2.22 MBq; Perkin Elmer, USA). That is, a coding region of AtPCS1 (AF085230) except for a termination codon was inserted into pENTR-D/TOPO (Invitrogen) according to the manufacturer's instructions, and then recombined into pDEST42 (Invitrogen). Except that AtPCS1-6xHis was expressed in BL21 cells (Novagen), and the purified protein was eluted in 0.25 M imidazole, a target protein was purified according to a method disclosed in Saavedra E, et al, (2005) Glycolysis in Entamoeba histolytica. Biochemical characterization of recombinant glycolytic enzymes and flux control analysis. FEBS J 272:1767-83.] After the purification, a buffer (0.25 M imidazole) including AtPCS1-6xHis was replaced with 0.1 M Tris (pH 8.0) using PD-10 column (GE Healthcare). A PC-synthesis reaction (100 µL) was performed at 37 °C for 60 minutes in a 0.1 M Tris solution (pH 8.0) containing 5 µg of AtPCS1-6xHis, 0.1 mM CdCl₂, 3 mM GSH and 30 µL of ³⁵S-GSH (60 uCi, 2.22 MBq; Perkin Elmer, USA). PC was separated using HPLC according to a method disclosed in Mendoza-Cozatl DG, et al, (2008) Identification of high levels of phytochelatins, glutathione and cadmium in the phloem sap of B. napus. A role for thiol-peptides in the long-distance transport of cadmium and the effect of cadmium on iron translocation. Plant J 54: 249-259, except that no formation of derivatives and PC elution were observed at 220 nm. Finally, the ³⁵S-PC₂ was resuspended in 0.1 M Tris (pH 8.0), 0.5 mM dithiothreitol (DTT) and 0.5 mM nonradioactive PC₂ (AnaSpec, CA, USA). To form a complex of PC₂ and As(III), As(III), PC₂ and DTT were mixed at a ratio of 2:1:1, and incubated at room temperature for 40 minutes.

### 1) Test on time dependency of PC₂-As transport in microsomal vesicle of yeast

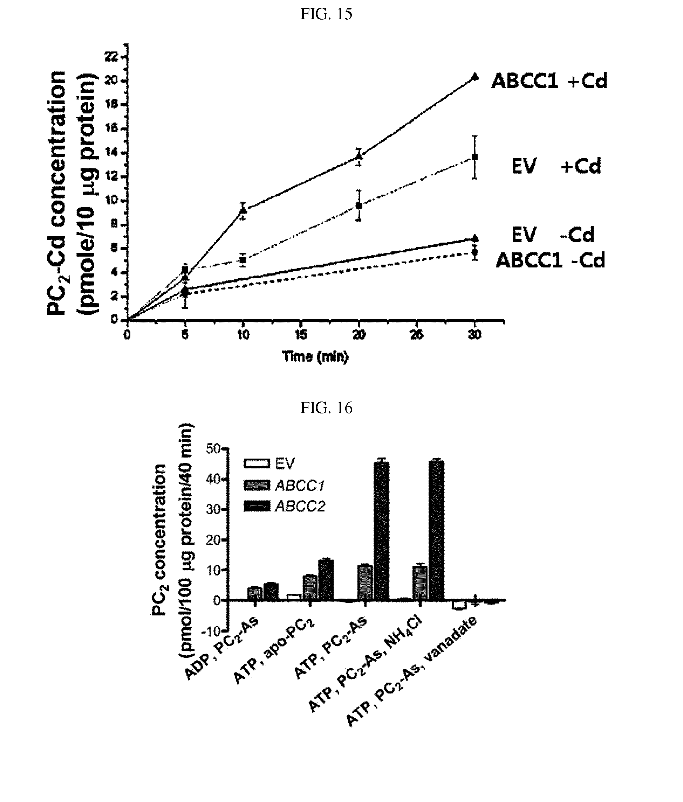

A test on time-dependent absorption of PC₂-As and PC₂-Cd into the microsomal vesicle of yeast was performed in a transporting buffer containing 25 µM PC₂ (4 mM ATP, 5 mM MgCl₂, 10 Mm creatine phosphate, 16 units/ml creatine kinase (Sigma), 1 mg/ml BSA, 100 mM KCl and 25 mM Tris-Mes (pH 7.4)). The results are shown in FIGS. 14 and 15. The PC₂-As and PC₂-Cd were effectively absorbed into the vesicles separated from the yeast cells that express AtABCC1 or AtABCC2 efficiently absorbed PC₂-As and PC₂-Cd in a time-dependent manner while the absorption amount from the separated vesicles from the void vector control was at a negligible level.

### 2) Test on comparison of transport activities of apo-PC₂ and PC₂-As and inhibitory activities of PC₂-As transportation by NH₄⁺ and vanadate

The transport activities comparison of apo-PC₂ and PC₂-As and the inhibitory activities of PC₂-As transportation by NH₄⁺ and vanadate are shown in FIG. 16. The inhibitory activities by vanadate and NH₄Cl were examined after these compounds were added respectively at concentrations of 1 mM and 5 mM into the solution used in the transportation analysis. In this case, a concentration of PC was 25 µM. From the results of comparison of the transport activities of apo-PC₂ and PC₂-As, it was revealed that the AtABCC1 and AtABCC2 more effectively transported PC₂-As complex, compared with the apo-PC₂. A transport activity ratio between apo-PC₂ and PC₂-As was shown to be different between other populations of the formed PC₂ due to variable stability of the PC₂-As complex. A difference in transport activity ratio between the apo-PC₂ and PC₂-As complexes was observed more clearly in the AtABCC2, compared with the AtABCC1.

Also, when a proton gradient vanished with NH₄⁺, the absorption of the PC₂-As complex into the yeast vesicle was not inhibited, as expected from the ABC-type transporter protein, but the transport activities were remarkably reduced in the presence of vanadate.

### 3) Test on transportation of arsenic (As) in a non-chelated form

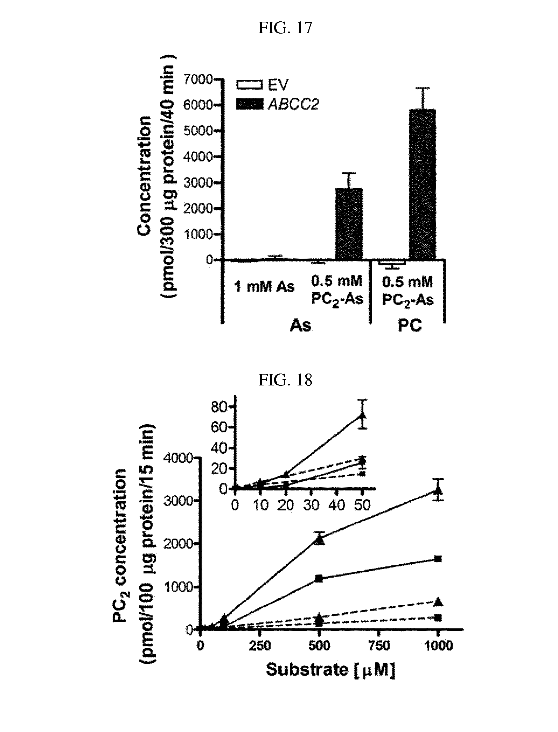

A test on whether the AtABCC2 transported arsenic (As) in a non-chelated form was performed using the microsomal vesicles of the SM7 cells expressing the AtABCC2. The results are shown in FIG. 17. Contents of PC₂ (right-hand bar) and arsenic (left-hand bar) transported were measured using 1 mM arsenite alone or in combination with 500 µM PC₂ prepared in Example 3 were measured. From the content of arsenite (As) measured using ICP-MS, it was revealed that arsenite was not transported by means of the AtABCC2 without using PC (FIG. 17). From the comparison of concentrations of PC₂ (right-hand bar) and arsenic (left-hand bar) transported into the vesicles, it could be also seen that two molecules of PC₂ were transport per arsenic molecule by the AtABCC2 (FIG. 17).

### 4) Concentration dependency on transport activities of PC₂-As and apo-PC₂

Concentration dependency of the vesicles separated from S. *cerevisiae* expressing AtABCC1 (square) or AtABCC2 (triangle) on transport activities of the PC₂-As (solid line) and apo-PC₂ (dotted line) complexes is shown in FIG. 18. Surprisingly, concentration-dependent PC₂-As absorption did not depend on conventional saturation kinetics in either the AtABCC1 or AtABCC2, but was observed in a pattern similar to a sigmoid curve (FIG. 18). An annex graph is an exploded version showing the results obtained in a low concentration of a substrate. Average ± standard error of estimation (obtained from three independent experiments, each of which was performed four times). The PC₂-As absorption activities were negligible in a PC₂ concentration of 10 µM or less, but potently promoted with an increase in PC concentration.

A concentration of PC could be measured to be 350 µM at the full width at half maximum of the absorption activities. Such a high concentration of PC shows somewhat low affinity of the AtABCC1 and AtABCC2 to PC. Interestingly, the concentration dependency on the apo-PC₂ complex was shown to be linear, and did not show a saturation curve until a concentration of the apo-PC₂ complex reached 1000 µM (FIG. 18).

### Example 4: Test on whether AtABCC1 and AtABCC2 transport PC₂-As into plant vacuole

To determine whether the AtABCC1 and AtABCC2 actually encode functional phytochelatin transporter proteins in a plant and measure relative contribution of the AtABCC1 and AtABCC2 in PC absorption, the transport activities of the intact vacuoles separated from the *atabcc1 atabcc2* double knock-out plant and the wild-type (WT) plant with respect to the PC₂-As and apo-PC₂ complexes were compared. ADP was used as the negative control instead of ATP. A concentration of PC was 250 µM.

Vacuoles were prepared from A. *thaliana* mesophyll protoplasts separated from an A. *thaliana* wild-type plant (Salk Genomic Analysis Laboratory (http://signal.salk.edu/)) using a method disclosed in Song WY, et al, (2003) Engineering tolerance and accumulation of lead and cadmium in transgenic plants. Nat Biotechnol 21:914-919 and Frangne N, et al, (2002) Flavone glucoside uptake into barley mesophyll and Arabidopsis cell culture vacuoles. Energization occurs by H+-antiport and ATP-binding cassette-type mechanisms. Plant Physiol 128:726-733. Research on transportation using the A. *thaliana* mesophyll vacuoles was conducted according to a method disclosed in the literatures regarding barley, and silicone oil poly(dimethylsiloxane-co-methylphenylsiloxane) 550 (Aldrich, http://www.sigmaaldrich.com) was used instead of AR200. Analysis of the apo-PC₂ included 200 µM PC₂ and 200 µM DTT; and analysis of PC₂-As included 200 µM PC₂, 400 µM As(III), and 200 µM DTT. In all the analyses, 50 nCi (1840 Bq) ³⁵S-labeled PC₂ and 50 nCi (1840 Bq) ³H₂O were used as tracers. A volume of the vacuole was measured using ³H₂O. A transportation rate was calculated in consideration of radioactivity absorbed 20 minutes after incubation with respect to radioactivity absorbed 2.5 minutes after incubation.

To measure a content of PC, *A*. *thaliana* wild-type and *abcc1 abcc2* double knock-out plants (Salk Genomic Analysis Laboratory (http://signal.salk.edu/)) were stratified at 4 °C for 2 days, and germinated in a 1/4 MS medium (Duchefa) according to a method disclosed in Sung DY, et al, (2009) ARS5 is a component of the 26S proteasome complex, and negatively regulates thiol biosynthesis and arsenic tolerance in Arabidopsis. Plant J 59, 802-813. The germinated plants were grown for 12 days, transferred to 1/4 MS media containing 50 and 100 µM potassium arsenate (K₂HAsO₄), and then exposed for 96 hours. Thereafter, the sum of accumulated amounts of PC was extracted and quantified using the HPLC-MS method disclosed in the literature. Average standard error of estimation (50 and 100 µM arsenic) in twice repeated experiments (0 µM arsenic) or three times repeated experiments.

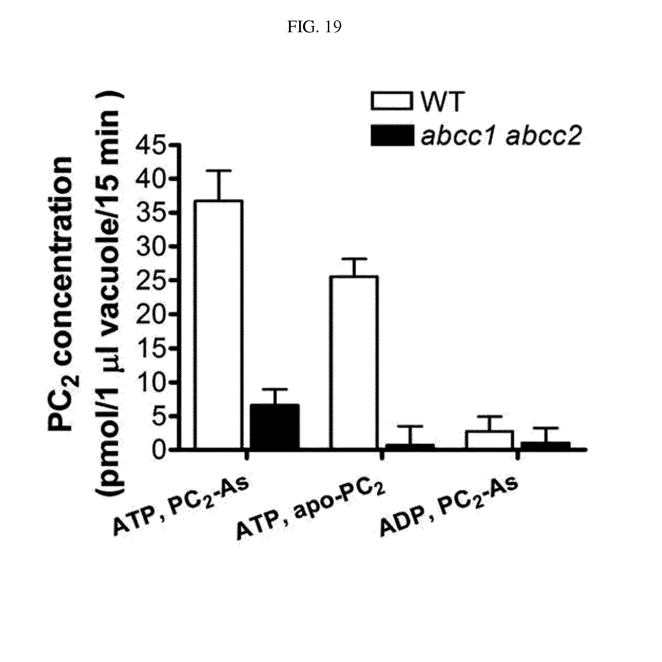

As shown in FIG. 19, the transport activities of the vacuoles with respect to the PC₂-As and apo-PC₂ complexes were drastically reduced to 85% and 95%, respectively, in the *atabcc1 atabcc2* double knock-out mutant plant, and thus the residual PC transport activities were observed at a very low level, which indicated that the two ABC transporter proteins are the most important PC and PC-bound transporter proteins.

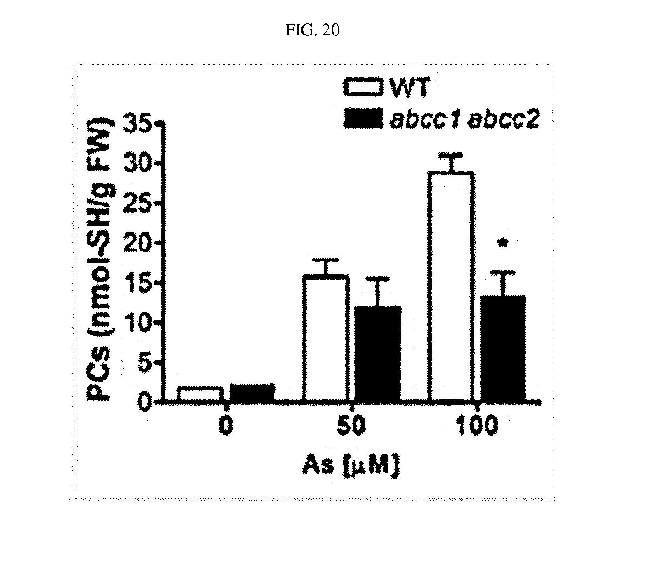

Also, when a total content of plant PC reflecting a pool of vacuoles and cytoplasm was measured, a content of phytochelatin combined through continuous arsenic exposure for 2 days was decreased in the *atabc1 atabc2* double knock-out plant (FIG. 20).

A difference between the wild-type plant and the mutant was more clearly observed at a high arsenic concentration at which increased production of phytochelatin was induced. Such observation corresponded to vacuolar phytochelatin transport kinetics observed in the vesicles isolated from yeast expressing two AtABCC transporter proteins. The homologues, AtABCC11 and AtABCC12, having high amino acid sequences similarity with the AtABCC1 and AtABCC2 proteins, might be able to play a role in a very low level of residual PC absorption.

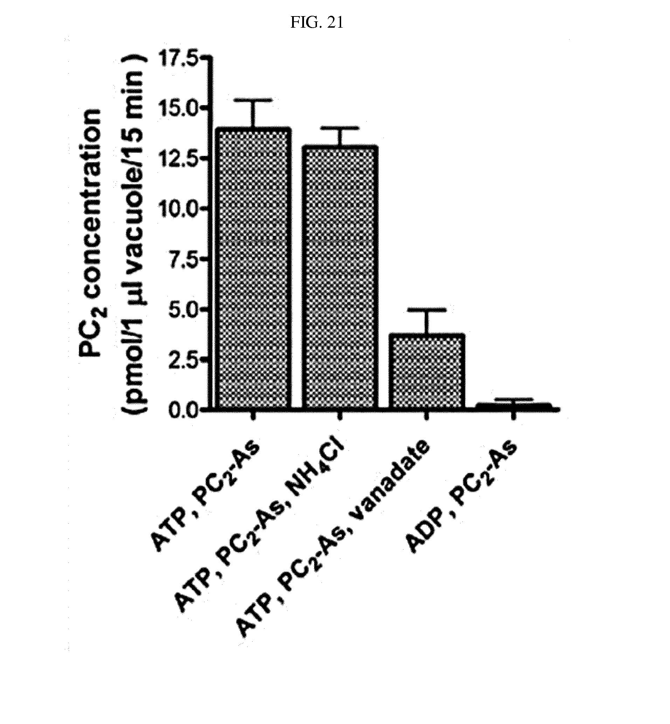

As observed in yeast, the PC transport of the vacuoles isolated from *A*. *thaliana* was not inhibited by NH₄⁺ (residual transport activity: 98±4%), but strongly inhibited by vanadate (residual transport activity: 25±7%) (FIG. 21), which was typical pattern to the ABC transporter proteins.

### Example 5: Test on whether expression of AtABCC1 and AtABCC2 is induced by arsenic treatment in A. thaliana

A 2-week-old wild-type *A*. *thaliana* seedling (Salk Genomic Analysis Laboratory (http://signal.salk.edu/cgi-bin/tdnaexpress)) was treated for 24 hours with 1/2 concentration MS media (Duchefa) to which 180 µM As(V) was added or not added. Thereafter, a total of RNA was extracted from the seedling using an RNeasy Plant Mini kit (Qiagen). To quantify transcripts of AtABCC1 and AtABCC2, quantitative realtime RT-PCR was performed using an SYBR kit (TAKARA, www.takara-bio.com/). Primers Salk_017431 LP (SEQ ID NO: 15) and Salk_017431 RP (SEQ ID NO: 16) were used to amplify the AtABCC1, and at the same time primers Salk_127425 LP (SEQ ID NO: 17) and Salk_127425 RP (SEQ ID NO: 18) were used to amplify the AtABCC2. The amplified samples were normalized with respect to a level of tubulin 8 (FIG. 22).

As shown in FIG. 22, it was revealed unlike PCS 1 which is induced by arsenic (As) in *A*. *thaliana,* AtABCC1 and AtABCC2's expression didn't increase by arsenic (As). Based on these results, it could be seen that the AtABCC1 and AtABCC2 were present in plant cells at all times to quickly respond to toxic metal (or metalloid) stress, instead of *de novo* synthesis of proteins, when exposed to a toxic heavy metal or metalloid.

### [Industrial Applicability]

The composition for transportation of phytochelatin according to the present invention is expected not only to be applicable to develop a crop in which the toxic substance present in edible parts, such as leaves, stems and fruits, of the plant is reduced in content, but also to be applied to inedible plants so as to contribute to developing a plant useful for phytoremediation or phytoextraction as it increases the accumulated amount of and resistance against arsenic, an arsenic compound and cadmium.

### [Sequence List Text]

SEQ. ID. NO. 1 (AtABCC1-s): CCGCAGAAATCCTCTTGGTCTTGATG

SEQ. ID. NO. 2 (AtABCC1-as): GTGAATCATCACCGTTAGCTTCTCTGG

SEQ. ID. NO. 3 (AtABCC1, 2F): ATCCGTGAACTCCGTTGGTATGTC

SEQ. ID. NO. 4 (AtABCC1, 2R): ACCACATGGTATGAAGTGATTGGC

SEQ. ID. NO. 5 (AtABCC2RT-F): AGCGTGCCAAAGATGACTCACACCAC

SEQ. ID. NO. 6 (AtABCC2RT-R): TACTTATCACGAAGAACACAACAGGG

SEQ. ID. NO. 7 (His3MX6-YHL035F):
GGGAACGGATCCCCTTATTATCCGAAATAATGGTTCATTTTGGGAAATCTGTTT
AGCTTGC

SEQ. ID. NO. 8 (His3MX6-YHL035R):
GCTCTAGGCCCCCACTGTCACGACACATACTATAAAATATACCGCGTTTCTGC
GCACTTAAC

SEQ. ID. NO. 9 (YLL015F): ACCAAAACCAAGAAAGAAAGTCGTTCC

SEQ. ID. NO. 10 (YLL015R): TGACTGAGAATACTGTTGTCATGTC

SEQ. ID. NO. 11 (YLL048CF): AAAACTGCTCCTGATCTCAAGCCAC

SEQ. ID. NO. 12 (YLL048CR): AATGTTCTCTCGAGCTTCCAAGGCC

SEQ. ID. NO. 13 (TaPCS1::cup1-1F):
ATAGATATTAAGAAAAACAAACTGTACAATCAATCAATCAATCATCACATAAA
ATGGAGGTGGCGTCGCTG

SEQ. ID. NO. 14 (TaPCS1::cup1-1R):
TTTAAAAATTAAAAACAGCAAATAGTTAGATGAATATATTAAAGACTATTCGTT
TCAAGTGAGCAGATTGTCGCAG

SEQ. ID. NO. 15 (Salk_017431 LP): TTCACTGTATCCATTGCCG

SEQ. ID. NO. 16 (Salk_017431 RP): TTACCGGCTTAGGTCTGCACTG

SEQ. ID. NO. 17 (Salk_127425 LP): GTGTTCGGGGTGCTATGTGAA

SEQ. ID. NO. 18 (Salk_127425 RP): CTGTAAAGGGAACATAAGGAC

<110>    POSTECH ACADEMY-INDUSTRY FOUNDATION

<120>    COMPOSITION FOR TRANSPORTING PHYTOCHELATIN

<130>    PCT01243

<150>    KR 10-2010-0106079
<151>    2010-10-28

<160>    18

<170>    KopatentIn 2.0

<210>    1
<211>    26
<212>    DNA
<213>    Artificial Sequence

<220>
<223>    AtABCC1-s


<400>    1
ccgcagaaat cctcttggtc ttgatg                                            26


<210>    2
<211>    27
<212>    DNA
<213>    Artificial Sequence

<220>
<223>    AtABCC1-as


<400>    2
gtgaatcatc accgttagct tctctgg                                           27


<210>    3
<211>    24
<212>    DNA
<213>    Artificial Sequence

<220>
<223>    AtABCC1,2F


<400>    3
atccgtgaac tccgttggta tgtc                                              24


<210>    4
<211>    24
<212>    DNA
<213>    Artificial Sequence

<220>
<223>    AtABCC1,2R


<400>    4
accacatggt atgaagtgat tggc                                              24

<210>	5
<211>	26
<212>	DNA
<213>	Artificial Sequence

<220>
<223>	AtABCC2RT-F

<400>	5
agcgtgccaa agatgactca caccac	26

<210>	6
<211>	26
<212>	DNA
<213>	Artificial Sequence

<220>
<223>	AtABCC2RT-R

<400>	6
tacttatcac gaagaacaca acaggg	26

<210>	7
<211>	61
<212>	DNA
<213>	Artificial Sequence

<220>
<223>	His3MX6-YHL035F

<400>	7
gggaacggat ccccttatta tccgaaataa tggttcattt tgggaaatct gtttagcttg	60

c	61

<210>	8
<211>	62
<212>	DNA
<213>	Artificial Sequence

<220>
<223>	His3MX6-YHL035R

<400>	8
gctctaggcc cccactgtca cgacacatac tataaaatat accgcgtttc tgcgcactta	60

ac	62

<210>	9
<211>	27
<212>	DNA
<213>	Artificial Sequence

<220>
<223>    YLL015F


<400>    9
accaaaacca agaaagaaag tcgttcc                                          27


<210>    10
<211>    25
<212>    DNA
<213>    Artificial Sequence

<220>
<223>    YLL015R


<400>    10
tgactgagaa tactgttgtc atgtc                                            25


<210>    11
<211>    25
<212>    DNA
<213>    Artificial Sequence

<220>
<223>    YLL048CF


<400>    11
aaaactgctc ctgatctcaa gccac                                            25


<210>    12
<211>    25
<212>    DNA
<213>    Artificial Sequence

<220>
<223>    YLL048CR


<400>    12
aatgttctct cgagcttcca aggcc                                            25


<210>    13
<211>    71
<212>    DNA
<213>    Artificial Sequence

<220>
<223>    TaPCS1::cup1-1F


<400>    13
atagatatta agaaaaacaa actgtacaat caatcaatca atcatcacat aaaatggagg      60

tggcgtcgct g                                                           71


<210>    14

<211>    76
<212>    DNA
<213>    Artificial Sequence

<220>
<223>    TaPCS1::cup1-1R


<400>    14
tttaaaaatt aaaaacagca aatagttaga tgaatatatt aaagactatt cgtttcaagt          60

gagcagattg tcgcag                                                          76


<210>    15
<211>    19
<212>    DNA
<213>    Artificial Sequence

<220>
<223>    Salk_017431 LP


<400>    15
ttcactgtat ccattgccg                                                       19


<210>    16
<211>    22
<212>    DNA
<213>    Artificial Sequence

<220>
<223>    Salk_017431 RP


<400>    16
ttaccggctt aggtctgcac tg                                                   22


<210>    17
<211>    21
<212>    DNA
<213>    Artificial Sequence

<220>
<223>    Salk_127425 LP


<400>    17
gtgttcgggg tgctatgtga a                                                    21


<210>    18
<211>    21
<212>    DNA
<213>    Artificial Sequence

<220>
<223>    Salk_127425 RP


<400>    18

ctgtaaaggg aacataagga c 21

## Claims

1. A composition for transportation of phytochelatin, comprising a DNA molecule encoding an ABCC (multidrug resistance-associated protein (MRP)) subfamily of ATP-binding cassette (ABC) transporter protein of a plant.

2. The composition according to claim 1, wherein the phytochelatin is in the form of a complex of phytochelatin with at least one selected from the group consisting of arsenic, cadmium, and a compound thereof.

3. The composition according to claim 1, wherein the DNA molecule is at least one selected from the group consisting of AtABCC1, AtABCC2, AtABCC11, AtABCC12, OsABCC1, and OsABCC3.

4. The composition according to claim 1, wherein the plant is selected from the group consisting of *Arabidopsis thaliana, Oryza sativa L., Nicotiana tabacum, Allium cepa, Daucus carota, Cucumis sativus, Brassica napus, Olea europaea, Ipomoea batatas, Solanum tuberosum, Brassica campestris* var. *pekinensis, Raphanus sativus, Lactuca sativa, Brassica oleracea* var. *italica, Petunia hybrida, Helianthus annuus, Brassica juncea* var. *integrifolia, Zoysia* spp., *Betula platyphylla* var. *japonica, Populus* spp., *Salix koreensis,* and *Betula schmidtii.*

5. A method of enhancing resistance of a plant against at least one selected from the group consisting of arsenic, cadmium and a compound thereof, comprising:
transforming a plant with the composition defined in any one of claims 1 to 4; and
transporting at least one selected from the group consisting of arsenic, cadmium and a compound thereof in the form of a complex with phytochelatin into a vacuole of the transformed plant.

6. A method of transforming a plant with the composition defined in any one of claims 1 to 4 and transporting phytochelatin, or a complex of the phytochelatin with at least one selected from the group consisting of arsenic, cadmium and a compound thereof into a vacuole of the transformed plant.
